# EUROPEAN PATENT SPECIFICATION

(11) **EP 3 282 989 B1**
(45) Date of publication and mention of the grant of the patent: **16.10.2019**
(21) Application number: 16718166.8
(22) Date of filing: 14.04.2016
(51) Int. Cl.: A61B 18/04

(54) **SURGICAL APPARATUS FOR ARGON BEAM COAGULATION**
CHIRURGISCHE VORRICHTUNG ZUR ARGONSTRAHLKOAGULATION
APPAREIL CHIRURGICAL POUR COAGULATION PAR FAISCEAU D'ARGON

(30) Priority: 15.04.2015 US 201562147627 P
(43) Date of publication of application: 21.02.2018
(73) Proprietor: ConMed Corporation, Utica, NY 13502 (US)
(72) Inventor: BARNES, Kelli Sue, Littleton, CO 80125 (US); TEMBURNI, Vishal, Englewood, CO 80111 (US); ROHLFING, Morgan, Leigh, Dublin, CA 94568 (US); RIFFELL, Daniel, Berthoud, CO 80513 (US); MOODY, Derek Lite, Needham, MA 02494 (US); BROWN, Brendan Ring, Okemos, MI 48864 (US)
(74) Representative: DREISS Patentanwälte PartG mbB
(86) International application number: PCT/US2016/027512
(87) International publication number: WO 2016/168449

(56) References cited:
- EP-A1- 1 090 597
- US-A- 6 039 736
- US-A1- 2005 171 528
- US-A1- 2010 125 267

## Description

### BACKGROUND OF THE INVENTION

The subject matter disclosed herein relates generally to surgical devices as well as to surgical techniques for electrothermal hemostasis.

### 2. Description of the Related Art

Electrosurgical techniques can be useful to achieve coagulation of fluids on lacerated tissue or like bleeding lesions. In argon beam coagulation (ABC) techniques, argon gas is directed onto the target tissue. The ABC technique also introduces electrical energy (e.g., current) at radio frequency (RF) into the argon gas. In this way, the argon gas clears blood and other fluids from the surface of the target tissue, thereby allowing the RF energy to interact directly with the tissue without the fluid diverting all or part of the electrical energy away from the target tissue.

The ABC technique has been more successful than other surgical techniques in achieving hemostasis in solid organ injury, like splenic injuries for example, and is especially valuable in treating patients with blood coagulation deficiencies. The ABC technique has been shown to result in reduced operative blood loss, recurrent bleeding, surgical time, and surgical plume. Conventional ABC surgical devices emit an argon gas jet with specific permanent features, such as area, diameter, cross-sectional area, and like geometries. However, if a feature of the argon gas jet is too large for the target tissue area, there is a potential of damage to healthy tissue and floating eschar. On the other hand, an argon gas jet that is too small for the target tissue area can require increased surgical time, which may lead to increased operative blood loss.

Accordingly, there is a need in the art for a surgical apparatus configured to allow an end user (e.g., a surgeon) to change the affected area of the target tissue during ABC techniques to lessen the risk of tissue damage while still achieving the aforementioned benefits of the ABC techniques.

Features related to the subject-matter of claim 1 are known from EP 1 090 597 A1.

### SUMMARY OF THE INVENTION

The invention is defined in appended independent claim 1, preferred embodiments are described in the dependent claims.

### BRIEF DESCRIPTION OF THE DRAWINGS

The present invention will be more fully understood and appreciated by reading the following Detailed Description in conjunction with the accompanying drawings. The accompanying drawings illustrate only typical embodiments of the disclosed subject matter and are therefore not to be considered limiting of its scope, for the disclosed subject matter may admit to other equally effective embodiments.

Reference is now made briefly to the accompanying drawings, in which:
FIG. 1 is a schematic diagram of an exemplary embodiment of an apparatus;
FIG. 2 is a perspective view of the front of a configuration not according to the invention in assembled form;
FIG. 3 is a perspective view of the back of the apparatus of FIG. 2 in exploded form;
FIG. 4 is an elevation view of the cross-section of the apparatus of FIG. 2;
FIG. 5 is an elevation view of the front of the apparatus of FIG. 2;
FIG. 6 is an elevation view of the back of the apparatus of FIG. 2;
FIG. 7 is a perspective view of the back of an example of a nozzle member;
FIG. 8 is an elevation view of the side of the nozzle member of FIG. 7;
FIG. 9 is an elevation view of the back of the nozzle member of FIG. 7;
FIG. 10 is a detail view of the elevation view of FIG. 9;
FIG. 11 is an elevation view of the front of the nozzle member of FIG. 7;
FIG. 12 is an elevation view of the cross-section of the nozzle member of FIG. 7 in a first position;
FIG. 13 is an elevation view of the cross-section of the nozzle member of FIG. 7 in a second position that is annularly offset from the first position;
FIG. 14 is a perspective view of the back of an example of a fluid connector;
FIG. 15 is an elevation view of the side of the fluid connector of FIG. 14;
FIG. 16 is an elevation view of the back of the fluid connector of FIG. 14;
FIG. 17 is an elevation view of the side of an example of an electrode;
FIG. 18 is an elevation view of the side of an example of an electrical connector;
FIG. 19 is an elevation view of the side of an example of a nozzle member;
FIG. 20 is an elevation view of the front of the nozzle member of FIG. 19;
FIG. 21 is an elevation view of the side of an example of a nozzle body of an alternative configuration not according to the invention;
FIG. 22 is an elevation view of the front of the nozzle body of FIG. 21;
FIG. 23 is a perspective view of an example of a nozzle body of an embodiment of the invention;
FIG. 24 is an elevation view of the side of an example of a nozzle body;
FIG. 25 is an elevation view of the side of an example of a nozzle body;
FIG. 26A is a front of the nozzle body of FIG. 25; and
FIG. 26B is a front of the nozzle body of FIG. 25.

Where applicable, like reference characters designate identical or corresponding components and units throughout the several views, which are not to scale unless otherwise indicated. Moreover, the embodiments disclosed herein may include elements that appear in one or more of the several views or in combinations of the several views.

### DETAILED DESCRIPTION

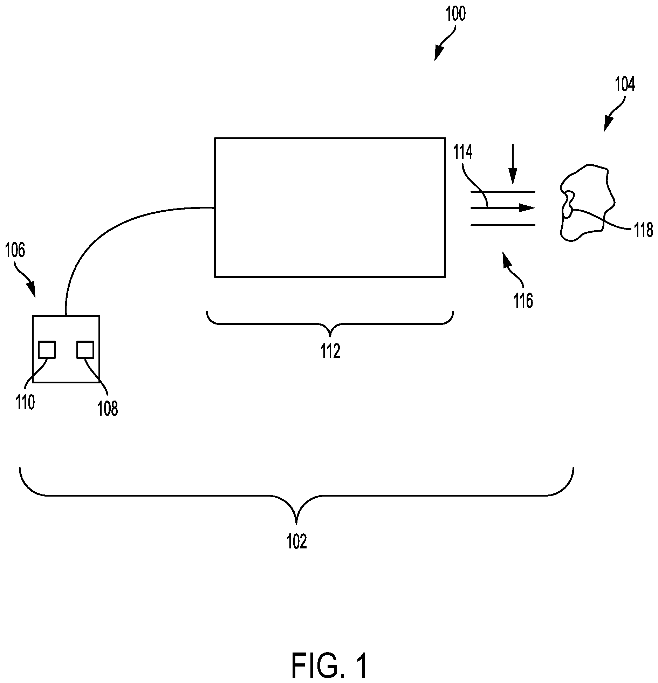

Referring now to the drawings, wherein like reference numerals refer to like parts throughout, FIG. 1 depicts a schematic diagram of an exemplary example of an apparatus 100. This example is part of a surgical unit 102 (also, "kit 102") that can effect hemostasis in a target 104 causing rapid coagulation in bleeding tissue. In addition to the apparatus 100, the surgical unit 102 may include a feed source 106 to provide, e.g., power and fluid to the apparatus 100. The feed source 106 may include a fluid delivery apparatus 108 and an electrosurgical generator 110 (also, "ESG 110"). The apparatus 100 includes a handpiece 112 that couples with the feed source 106. The handpiece 112 is configured to generate a jet 114 with a feature 116 that defines dimensions of a coagulation area 118 on the target 104. In use, a surgeon can manipulate the handpiece 112 to direct the jet 114 onto the target 104 to ensure proper hemostasis during a surgical procedure. The jet 114 can comprise an ionized fluid, preferably gas (e.g., argon), although this disclosure does not foreclose use of liquids in connection with the embodiments disclosed herein.

Broadly, the handpiece 112 is configured to modify the feature 116 of the jet 118. Examples of the feature 116 can include area, diameter, cross-sectional area, geometry, shape, and like characteristics that can affect the size of the coagulation area on the target 104. Such modifications may, for example, modify the cross-sectional area of the jet 114 so as to increase and decrease the size of the coagulation area on the target 104.

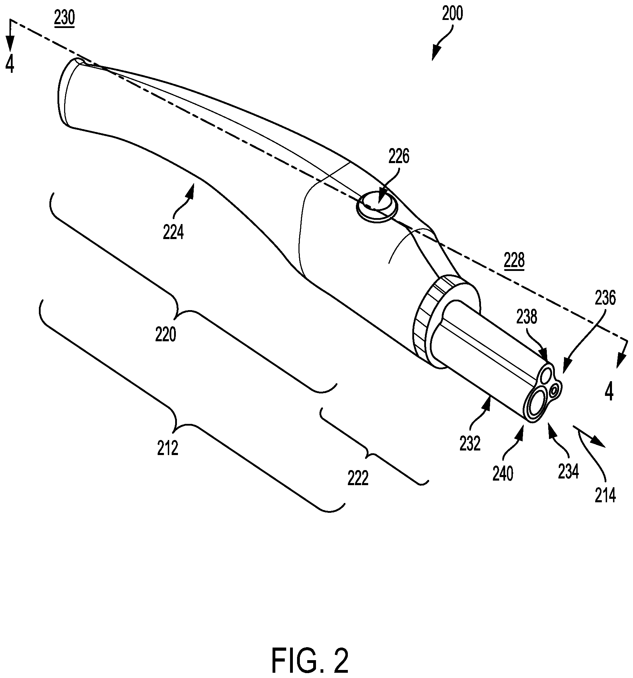

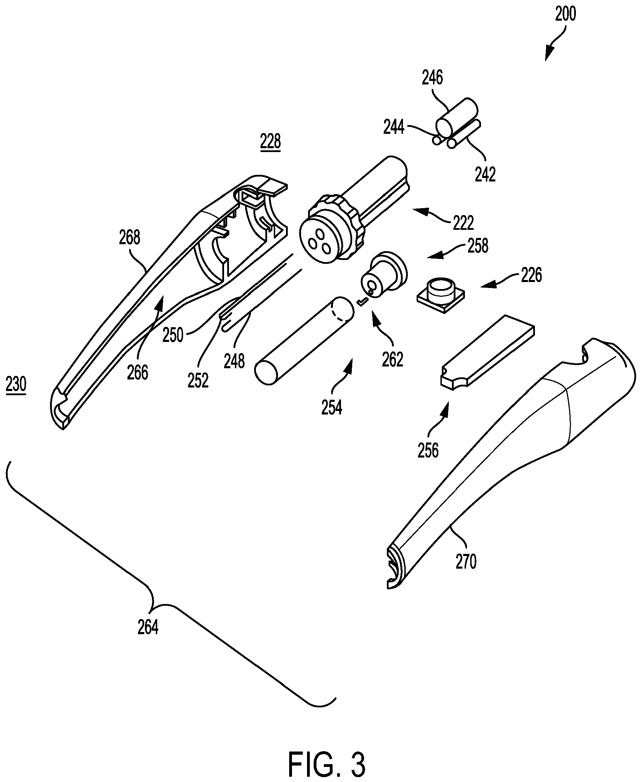

FIGs. 2 and 3 illustrate a perspective view of an exemplary example of an apparatus 200. FIG. 2 shows the apparatus 200 from the front and in assembled form. FIG. 3 shows the apparatus 200 from the back and in exploded form.

Turning first to FIG. 2, the handpiece 212 can include one or more constituent members (e.g., a body member 220 and a nozzle member 222). The body member 220 can have a handle portion 224 through which penetrates an actuator 226. The body member 220 has a pair of ends (e.g., a first or nozzle end 228 and a second or inlet end 230). The nozzle member 222 is disposed on the first end 228. In one implementation, the nozzle member 222 can have a nozzle body 232 that is arranged with a dispensing feature, identified generally by the numeral 234. The dispensing feature 234 can be configured to define the feature 216 of the jet 214. In one example, the dispensing feature 234 embodies one or more bores (e.g., a first bore 236, a second bore 238, and a third bore 240) that form an exit aperture of different geometry relative to one another.

The handle portion 224 can form a landing region on the handpiece 212. This landing region can be contoured for a surgeon to comfortably grasp by hand in a manner that can locate the actuator 226 for actuation by one of the fingers and/or thumb. In use, the surgeon can manipulate the handpiece 212 much like a pen or pencil for maximum dexterity and feel during the surgical procedure. Depressing the actuator 226 activates the handpiece 212 to generate the jet 214.

FIG. 3 shows the apparatus 200 in exploded form to facilitate discussion of additional features and components of the device. Starting at the nozzle end 230 of the handle portion 224, the nozzle member 222 can include one or more inserts (e.g., a first insert 242, a second insert 244, and a third insert 246), one each sized and configured to fit into one of the corresponding bores 236, 238, 240 (FIG. 2). The inserts 242, 244, 246 outfit the nozzle body 232 to resist the high temperatures of the ionized fluid, preferably leveraging materials like ceramics and/or composites thereof. The nozzle member 222 can also include one or more electrodes (e.g., a first electrode 248, a second electrode 250, and a third electrode 252). The electrodes 248, 250, 252 are configured to conduct electrical charge into the bores 236, 238, 240. This electrical charge is necessary to ionize the fluid, creating plasma to effectuate hemostatis and coagulation of the bleeding tissue during operation of the device by the surgeon in the surgical procedure.

The handpiece 212 can include several ancillary components that facilitate operation of the device. These ancillary components may include a fluid distribution system 254 (also, "fluid system 254") and electronics 256. The fluid system 254 can include a fluid connector 258 that can couple with the nozzle body 232 and with a fluid supply line 260. The fluid connector 258 can be configured with a primary bore or lumen to allow fluid to flow into one or more of the bores 236, 238, 240. In one example, the fluid connector 258 can also conduct electrical current to the nozzle body 232 to energize the electrodes 248, 250, 252. This feature may require use of an electrical connector 262. In use, the fluid connector 258 may have a secondary bore or lumen to receive the electrical connector 262. However, this disclosure does contemplate myriad other constructions for which the fluid connector 258 can conduct such electrical current to energize the electrodes 248, 250, 252.

The electronics 256 can be configured to generate a signal in response to contact from the actuator 226. Printed circuit boards, chip carriers, and like circuitized substrates may be useful to generate this signal. This substrate can include at least one planar surface (or two opposing planar surfaces) on which electronic components such as semiconductor chips, resistors, capacitors, modules, etc. may be mounted. One or more chip carriers may be mounted to the printed circuit board, each chip carrier including one or more chips mounted to a chip carrier substrate, utilizing wire-bond or solder reflow technologies, for example. Circuit paths for the electronic components are typically provided by forming conductive lines, or traces, on the planar surface of the circuit board. The traces may extend from pads surrounding the electronic components to thru-holes in the circuit board (e.g., vias), or may extend to another component mounted on the board. The traces may extend to conductive pads at the edge of the circuit board, to which connection lead wires may be soldered or otherwise connected.

Referring back to FIG. 3, the body member 220 can form an enclosure 264 with an inner cavity 266 to receive one or more of the components of the handpiece 212 denoted above. The enclosure 264 can have a bifurcated configuration with one or more enclosure pieces (e.g., a first enclosure piece 268 and a second enclosure piece 270). This configuration arranges the enclosure pieces 268, 270 to each form a portion of the inner cavity 266 in assembled form. In one implementation, the enclosure pieces 268, 270 can be made from molded plastics for cost and manufacturing efficiencies, although there is nothing to prevent manufacture by way of machining and like techniques.

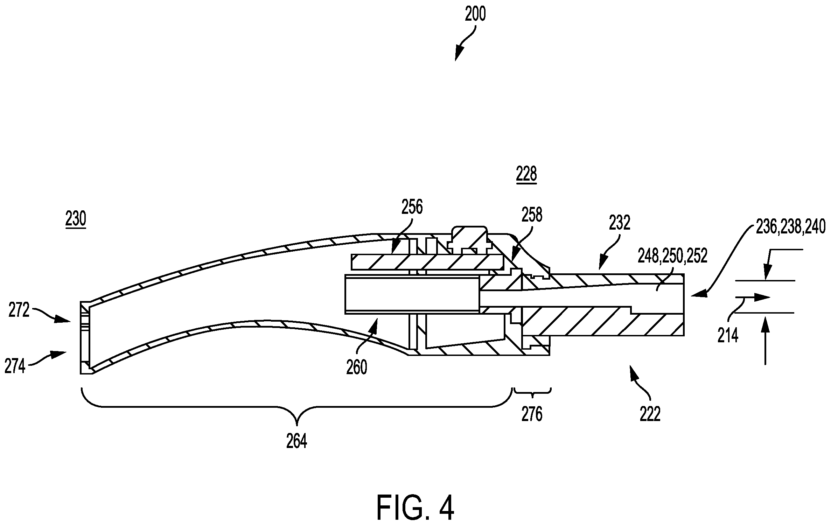

FIG. 4 illustrates an elevation view of the cross-section of the apparatus 200 taken at line 4-4 of FIG. 2. Each of the ends 228, 230 may be configured to allow access to the inner cavity 266 of the enclosure 264. At the inlet end 230, the enclosure 264 may include a pair of supply openings (e.g., a first supply opening 272 and a second supply opening 274). The nozzle end 228 of the enclosure 264 can form an interface 280 with an axis 278. The interface 280 retains each of the nozzle member 222 and the fluid connector 258 in a position to facilitate flow of fluid through the nozzle body 232. In one implementation, the interface 280 can be configured to allow the nozzle body 234 to translate, preferably to rotate about the axis 278. This configuration allows the surgeon to selectively align one of the bores 236, 238, 240 on the nozzle body 232 with the primary bore on the fluid connector 258. In turn, the jet 214 will assume the feature 216 that corresponds with the bore 236, 238, 240, as contemplated herein.

The supply openings 272, 274 may accommodate various conduits from the feed source 106 (FIG. 1). These conduits can include electrical cabling to supply power and related signals to electronics 256 via the first supply opening 272. The conduits may also include the fluid supply line 260, which can extend out of the enclosure 264 via the second supply opening 274. In one implementation, the apparatus 200 may be outfit with one or more suitably fashioned connectors disposed in and/or as part of the enclosure 264 at the supply openings 272, 274. These connectors may be configured to couple with the fluid supply line 260 (and electrical cabling extending from the electronics 256) on one side internal to the inner cavity 266. In use, the connectors can also be configured to couple with other cables and conduits on one side external to the enclosure 264.

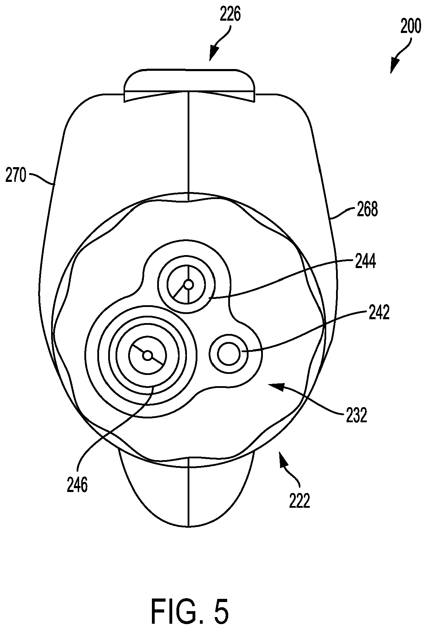

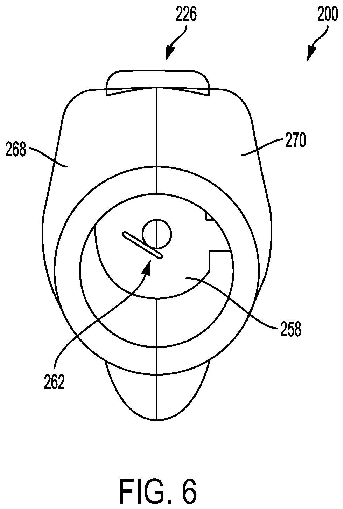

FIGs. 5 and 6 illustrate an elevation view of the apparatus 200 from the front (FIG. 5) and back (FIG. 6), respectively.

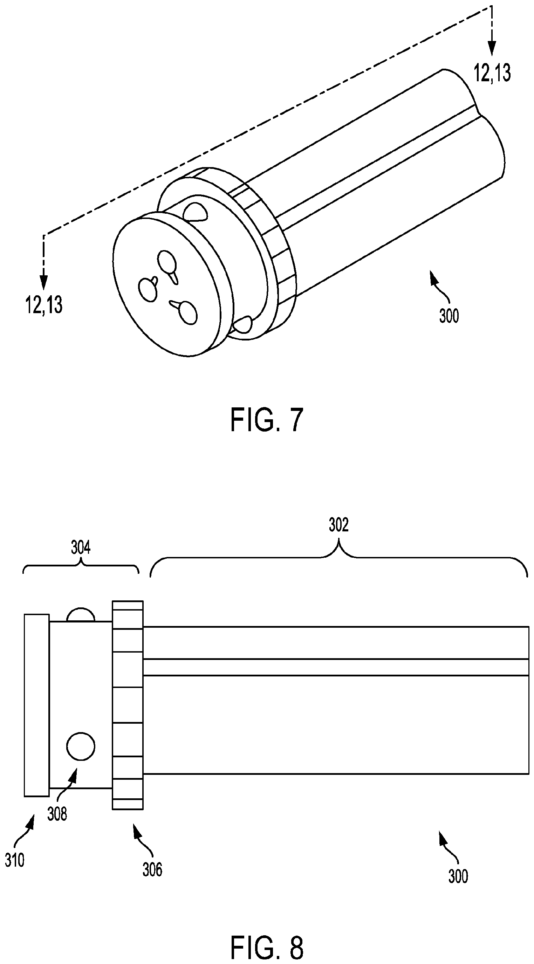

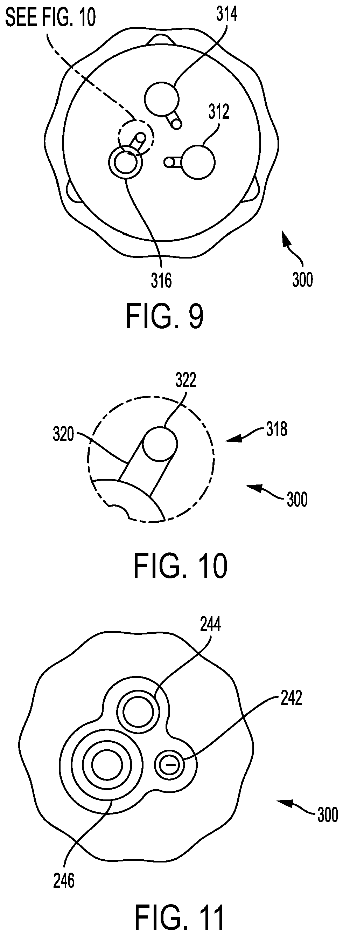

FIGs. 7, 8, 9, 10, 11, 12, and 13 illustrate various views an example 300 of the nozzle body 232. The nozzle body 300 includes a barrel section 302 and a drum section 304. In the drum section 304, the nozzle body 300 can include a knob member 306, one or more boss members 308, and a shoulder member 310. As best shown in FIG. 9, one end of the nozzle body 300 can include a plurality of openings (e.g., a first opening 312, a second opening 314, and a third opening 316) that terminate the bores 236, 238, 240. This end also includes an electrode feature 318 that is configured to receive, support, and position the electrodes 248, 250, 252 in the corresponding bores 236, 238, 240. The electrode feature 318 can include a slotted portion 320 and bore portion 322. The slotted portion 320 can adjoin the openings 312, 314, 316.

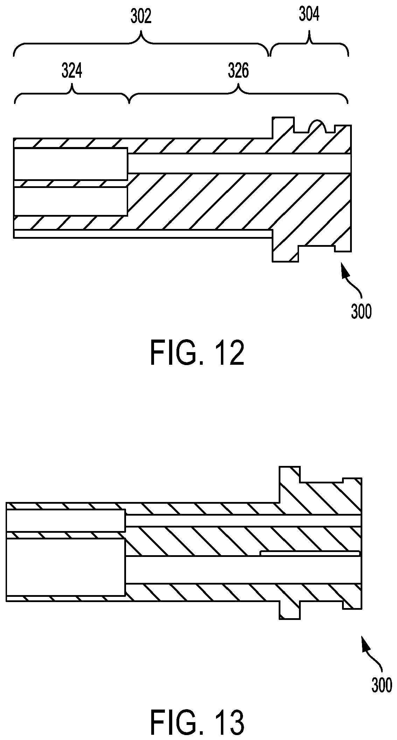

In FIGs. 12 and 13, the barrel section 302 can define a distal bore section 324 and a proximal bore section 326 for each of the bores 236, 238, 240. As shown, the proximal bore section 326 may extend into the drum section 304. Dimensions for the forward bore section 324 can be configured to receive inserts 242, 244, 246.

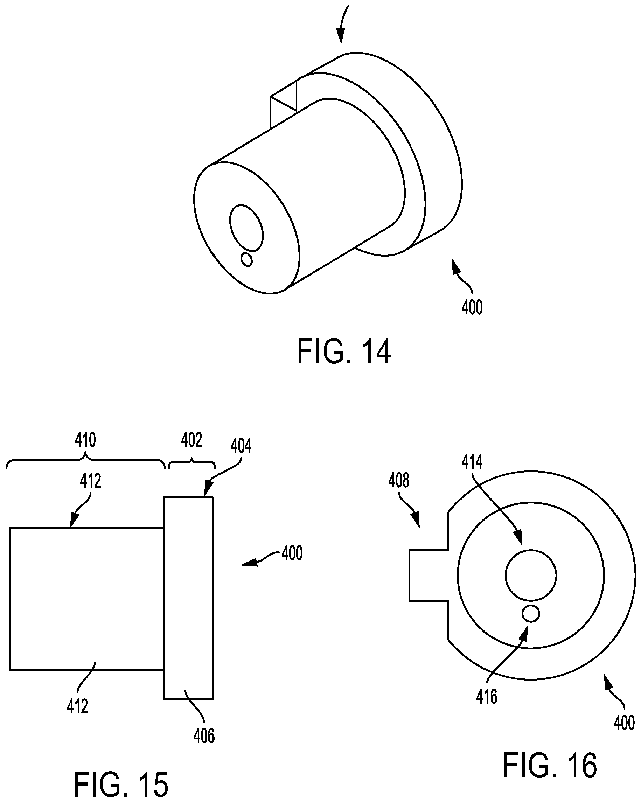

FIGs. 14, 15, and 16 illustrate various views of an example 400 of the fluid connector 258. The fluid connector 400 includes a nozzle section 402 that forms a shoulder 404 having an outer peripheral surface 406 of generally annular and/or cylindrical shape. The shoulder 404 can form a key member 408. In use, the key member 408 can engage part of the enclosure 264 to align the fluid connector 400 in the correct position for use in the handpiece 212. The shoulder 404 can abut a reduced diameter section 410. The reduced diameter section 410 also has an outer peripheral surface 412 of generally annular and/or cylindrical shape. The shape of the surface 412 may be either of constant outer dimensions (e.g., diameter) or differing outer dimensions (e.g., diameter), as desired.

The fluid connector 400 can include one or more bores or lumen (e.g., a primary bore or lumen 414 and a secondary bore or lumen 416). The primary bore 414 can penetrate into the material of the fluid connector 400, often forming a flow path for fluid to traverse through the fluid connector 400 from one end to the other end. The secondary bore 416 can also penetrate into the material of the fluid connector 400. In one implementation, the secondary bore 416 can be configured to receive the electrical connector 262. The connector 258 can form a seal with the nozzle member and, in some implementations, may be configured to accommodate a resilient member (e.g., gasket, o-ring) made of compressible material to form the seal.

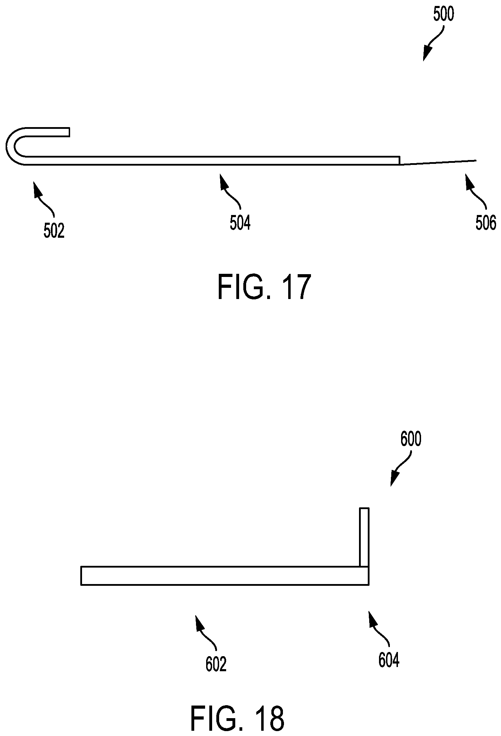

FIG. 17 illustrates an elevation view of an example 500 of the electrodes 248, 250, 252. The electrode 500 can have a bent end 502 and an elongate body 504 that terminates at a tapered end 506. The bend end 502 can be configured to insert into the electrode feature 318 with the elongate body 504 being positioned in the bore 236, 238, 240.

FIG. 18 illustrates an elevation view of an example 600 of the electrical connector 262. The electrode 600 can have an elongate body 602 that terminates at one end in a radially extending member 604. The elongate body 602 can extend into the secondary bore 416 on the fluid connector 400. With the elongate body 602 in positon on the fluid connector 400, the radially extending member 604 can be configured to contact the electrode 500 to conduct electrical current.

The electrode may also be a conductive tube electrode. A hollow conductive tube electrode can be composed of stainless steel or other suitable like material and comprises a sharpened outer edge such that the tube functions as an electrode. Radio frequency energy is supplied to the conductive tube via a multi-strand wire. Non-insulated strands from the multi-strand wire are secured to the conductive tube. The wire extends to an electrosurgical generator via a connector. An insulator is secured around the conductive tube for protection. Further, flexible tubing can be secured around the conductive tube and extend the length of the handpiece.

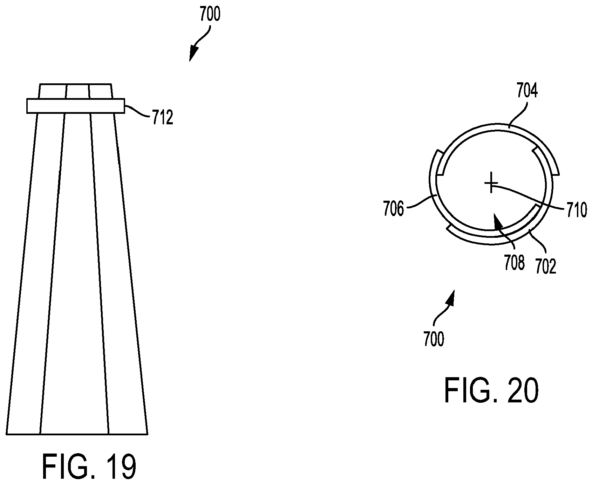

FIGs. 19 and 20 illustrate various views of an example 700 of the nozzle body 232. The nozzle body 700 includes one or more interleaved members (e.g., a first interleaved member 702, a second interleaved member 704, and a third interleaved member 706). The members 702, 704, 706 form a bore 708 with an axis 710. The device can also include a moveable member 712 that can translate along the axis 710 and along the interleaved members 702, 704, 706. Examples of the moveable member 712 can embody an annular ring that circumscribes the axis 710. The annular ring can have fixed dimensions (e.g., fixed diameter) so that, in one implementation, movement of the movable member 712 along the axis 710 will cause the members 702, 704, 706 to move relative to one another to change the size of the bore 708. The device may include a biasing element (not shown) or like structure to maintain tension and or force on the members 702, 704, 706, whether to maintain the members 702, 704, 706 in an open configuration or a closed configuration, as desired.

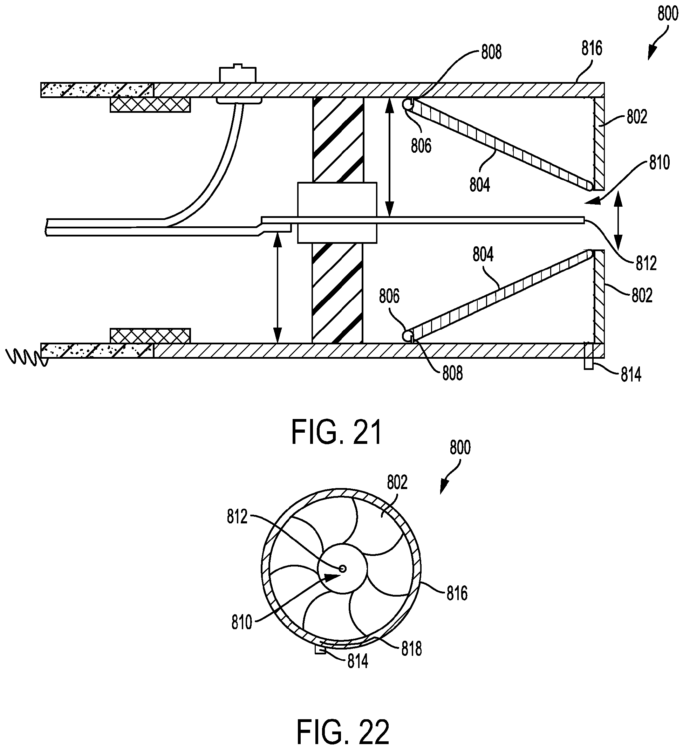

FIGs. 21 and 22 illustrate an example 800 of the nozzle body 232 of an alternative example. Referring first to FIG. 22, there is shown an elevation view of the front of a nozzle body 800 of FIG. 21. In the depicted example, the nozzle body 800 comprises a rigid outer barrel 816 having a lever 814 slidably disposed within a track 818 extending along the circumference of the outer barrel 816. The rigid outer barrel 816 further comprises an adjustable diaphragm having a plurality of blades 802 wherein sliding the lever 814 in the track 818 moves the blades 802 of the diaphragm from a first position to a second position. The blades 802 define a single central bore 810 that extends into the outer barrel 816. In the first position, the blades 802 define a central bore 810 with a first diameter. In the second position, the blades 802 define a central bore 810 having a second diameter different from the first diameter.

Referring now to FIG. 21, there is shown an elevation view of the side of an example of a nozzle member 800 of an alternative example. The blades 802 of the diaphragm are rigidly fixed to an inner nozzle 804 within the outer barrel 816. The inner nozzle 804 is also connected to a hinge 806 on the outer barrel 816. Sliding the lever 814 moves the blades 802, which ultimately cause the inner nozzle 804 to rotate about the hinge 804. The inner nozzle 804 directs the jet of argon gas out of the outer barrel 816 through the central bore 810.

A compressible member 808 is attached between the outer barrel 816 and the inner nozzle 804 near the hinge 806. The compressible member 808 provides a seal that aids in directing argon gas flow through the inner nozzle 804 and out the central bore 810. An electrode 812 is centered in the nozzle body 800 and is secured in place via a plastic overmold or other similar processes.

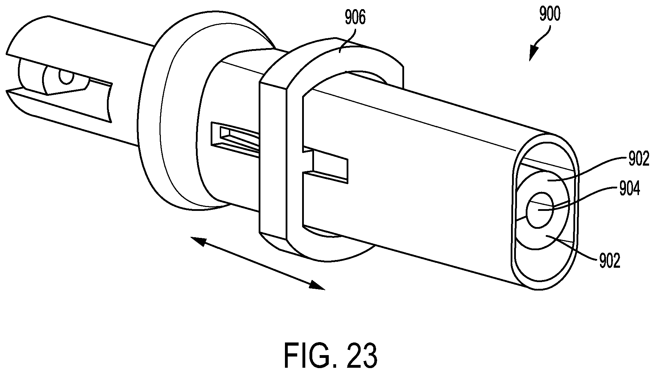

FIGs. 23, 24, 25, 26A, and 26B illustrate an example 900 of the nozzle body 232 of an embodiment according to the invention. Referring first to FIG. 23, there is shown a perspective view of the nozzle body 900. In the depicted embodiment, the nozzle body 900 comprises a tubular inner nozzle split into a pair of channel members 902. The channel members 902 define a central bore 904 extending through the inner nozzle. As will be discussed in detail below, a sliding collar 906 on the nozzle body 900 moves the channel members 902 relative to each other from a first position to a second position. In the first position, the central bore 904 has a first diameter and in the second position, the central bore 904 has a second diameter different from the first diameter.

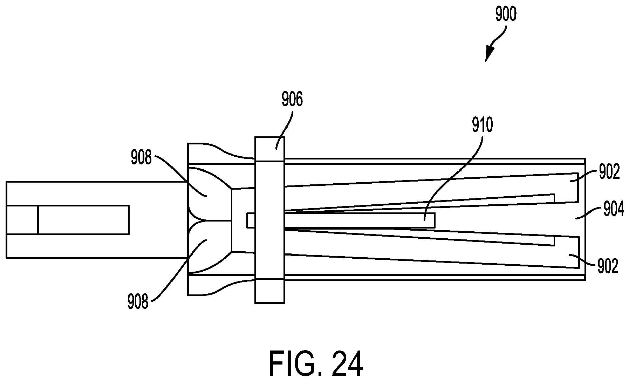

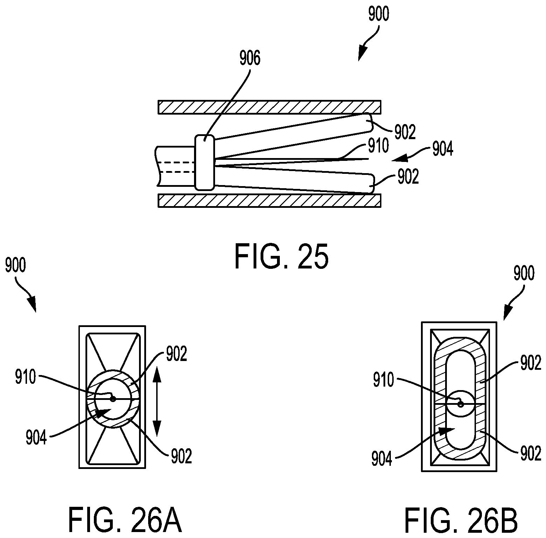

Referring now to FIG. 24 and 25, there are shown elevation views of the side of an example of a nozzle body 900. The channel members 902 within the nozzle body 900 are connected at a hinge 908 allowing the channel members 902 to move relative to each other. The hinge 908 is biased such that it forces the channel members 902 apart. However, the collar 906 can be moved along the nozzle body 900 to force the channel members 902 towards each other, forming a hollow cylindrical shape. In the depicted embodiment, an electrode 910 is positioned at the center of the nozzle body 900 and can be secured in place using a plastic overmold or like processes. Thus, when the channel members 902 are moved together or apart, the electrode 910 remains positioned in the center of the jet of argon gas flowing through the nozzle body 900.

Referring now to FIGs. 26A and 26B, there are shown elevation views of the front of a nozzle body 900 of FIG. 25. In FIG. 26B, the collar 906 is positioned closer to the hinge 908 such that the channel members 902 are forced apart. In this position, the channel members 902 define a central bore with a first diameter. In FIG. 26A, the collar 906 is moved away from the hinge 906 along the nozzle body 900 such that the channel members 902 are forced together. In this position, the channel members 902 define a central bore with a second diameter which is smaller than the first diameter. Thus, sliding the collar 906 along the nozzle body 900 alters a feature of the jet of argon gas emitted by the nozzle body 900. In use, the collar 906 can be easily moved back and forth along the nozzle body 900 to change the feature of the jet of argon gas as needed.

While embodiments of the present invention has been particularly shown and described with reference to certain exemplary embodiments, it will be understood by one skilled in the art that various changes in detail may be effected therein without departing from the scope of the invention as defined by claims that can be supported by the written description and drawings.

## Claims

1. A surgical apparatus (200) comprising:
a handpiece (212) configured to modify a feature (216) of a jet (214) of argon gas; wherein the feature (216) defines a cross-sectional area of the jet (214);
wherein the handpiece (212) comprises a nozzle member (222) with one or more bores (236, 238, 240) configured to direct argon gas as the jet (114) and one or more electrodes (248, 250, 252) configured to conduct electrical charge into the jet (114);
**characterized in that** the apparatus further comprises:
a collar (906) around the nozzle member (222);
wherein sliding the collar (906) along the nozzle member (222) changes the feature (116) of the jet (114) of argon gas;
wherein the nozzle member (222) comprises a pair of hingedly connected channel members (902);
wherein the channel members (902) are biased apart such that sliding the collar (906) along the nozzle member (222) forces the channel members (902) toward each other thereby changing the feature (216) of the jet (214) of argon gas.

2. The apparatus (200) of claim 1, further comprising one or more temperature resistant inserts (242, 244, 246), each insert sized and configured to fit within each bore (236, 238, 240).

3. The apparatus (200) of claim 1, further comprising an enclosure (264) that couples with the nozzle member (222), wherein the nozzle member (222) can rotate relative to the enclosure (264) to change the feature (216) of the jet (214) of argon gas.

4. The apparatus (200) of claim 1, wherein each of the bores (236, 238, 240) has a different diameter compared to the others.

5. The apparatus (200) of claim 1, further comprising a plurality of blades (802) coupled to the nozzle member (222) such that the blades (802) can rotate relative to the nozzle member (222) to change the feature (216) of the jet (214) of argon gas.

6. The apparatus (200) of claim 1, further comprising a slidable lever (814) on the nozzle member (222) configured to rotate the blades (802).

7. The apparatus (200) of 1, wherein the nozzle member (222) comprises a plurality of interleaved members (702, 704, 706) that move relative to one another when the collar (906) slides along the nozzle member (222).

## Patentansprüche

1. Chirurgische Vorrichtung, umfassend:
ein Handstück (212), das dazu dient, ein Merkmal (216) eines Argongasstrahls (214) zu modifizieren, wobei das Merkmal (216) eine Querschnittsfläche des Strahls (214) definiert
und das Handstück (212) ein Düsenelement (222) mit einer oder mehreren Öffnungen (236, 238, 240) umfasst, die dazu dienen, Argongas als Strahl (114) auszurichten, und eine oder mehrere Elektroden (248, 250, 252), die elektrische Ladung in den Strahl (114) leiten,
**dadurch gekennzeichnet, dass** die Vorrichtung zudem Folgendes umfasst:
eine um das Düsenelement (222) angeordnete Manschette (906),
wobei das Merkmal (116) des Argongasstrahls (114) verändert wird, wenn die Manschette (906) entlang dem Düsenelement (222) geschoben wird;
wobei das Düsenelement (222) ein Paar gelenkig miteinander verbundener Kanalelemente (902) umfasst,
wobei die Kanalelemente (902) auseinandergedrückt werden, so dass sie durch Schieben der Manschette (906) entlang dem Düsenelement (222) zueinander gedrückt werden, wodurch das Merkmal (216) des Argongasstrahls (214) verändert wird.

2. Vorrichtung (200) nach Anspruch 1, zudem umfassend einen oder mehrere temperaturbeständige Einsätze (242, 244, 246), wobei jeder Einsatz so bemessen und geformt ist, dass er in jede der Öffnungen (236, 238, 240) passt.

3. Vorrichtung (200) nach Anspruch 1, zudem umfassend ein Gehäuse (264), das mit dem Düsenelement (222) verbunden ist, wobei das Düsenelement (222) in Bezug auf das Gehäuse (264) drehbar ist, wodurch das Merkmal (216) des Argongasstrahls (214) verändert werden kann.

4. Vorrichtung (200) nach Anspruch 1, wobei jede der Öffnungen (236, 238, 240) im Vergleich zu den anderen Öffnungen einen unterschiedlichen Durchmesser aufweist.

5. Vorrichtung (200) nach Anspruch 1, zudem umfassend eine Vielzahl von Blättern (802), die mit dem Düsenelement (222) verbunden sind, so dass die Blätter (802) in Bezug auf das Düsenelement (222) drehbar sind, wodurch das Merkmal (216) des Argongasstrahls (214) verändert werden kann.

6. Vorrichtung (200) nach Anspruch 1, zudem umfassend einen an dem Düsenelement (222) angeordneten schiebbaren Hebel (814), der dazu dient, die Blätter (802) zu drehen.

7. Vorrichtung (200) nach Anspruch 1, wobei das Düsenelement (222) eine Vielzahl sich überlappender Elemente (702, 704, 706) umfasst, die sich relativ zueinander bewegen, wenn die Manschette (906) entlang dem Düsenelement (222) gleitet.

## Revendications

1. Instrument chirurgical (200) comprenant:
une pièce à main (212) configurée de manière à modifier une caractéristique (216) d'un jet (214) d'argon; dans lequel la caractéristique (216) définit une section transversale du jet (214);
dans lequel la pièce à main (212) comprend un corps de buse (222) avec un ou plusieurs alésage(s) (236, 238, 240) configuré(s) pour diriger l'argon à la manière du jet (114) et une ou plusieurs électrodes (248, 250, 252) configurées pour conduire une charge électrique dans le jet (114);
**caractérisé en ce que** l'instrument comprend en outre:
un collier (906) autour du corps de buse (222);
dans lequel le glissement du collier (906) le long du corps de buse (222) modifie la caractéristique (116) du jet (114) d'argon;
dans lequel le corps de buse (222) comprend une paire d'éléments de canal (902) reliés par une charnière;
dans lequel les éléments de canal (902) sont orientés de manière à ce que le glissement du collier (906) le long du corps de buse (222) force les éléments de canal (902) l'un vers l'autre, changeant de ce fait la caractéristique (216) du jet (214) d'argon.

2. Instrument (200) selon la revendication 1, comprenant en outre un ou plusieurs insert(s) résistant à la température (242, 244, 246), chaque insert étant dimensionné et configuré de manière à s'adapter dans chaque alésage (236, 238, 240).

3. Instrument (200) selon la revendication 1, comprenant en outre une enceinte (264) qui s'accouple avec le corps de buse (222), dans lequel le corps de buse (222) peut tourner par rapport à l'enceinte (264) afin de modifier la caractéristique (216) du jet (214) d'argon.

4. Instrument (200) selon la revendication 1, dans lequel chacun des alésages (236, 238, 240) a un diamètre différent comparé aux autres.

5. Instrument (200) selon la revendication 1, comprenant en outre une pluralité de lames (802) accouplées au corps de buse (222) de manière à ce que les lames (802) puissent tourner par rapport au corps de buse (222) afin de modifier la caractéristique (216) du jet (214) d'argon.

6. Instrument (200) selon la revendication 1, comprenant en outre un levier pouvant coulisser (814) sur le corps de buse (222) configuré de manière à faire tourner les lames (802) .

7. Instrument (200) selon la revendication 1, dans lequel le corps de buse (222) comprend une pluralité d'éléments intercalés (702, 704, 706) qui se déplacent l'un par rapport à l'autre lorsque le collier (906) glisse le long du corps de buse (222).
